(19) **Europäisches Patentamt**

**European Patent Office**

**Office européen des brevets**

(11) **EP 0 912 445 B1**

(12) **FASCICULE DE BREVET EUROPEEN**

(45) Date de publication et mention
de la délivrance du brevet:
**15.10.2003 Bulletin 2003/42**

(21) Numéro de dépôt: **97929045.9**

(22) Date de dépôt: **04.07.1997**

(51) Int Cl.[7]: **C02F 1/36**, C02F 1/32,
C02F 1/48, C02F 1/72,
A61L 2/02, B01J 19/10

(86) Numéro de dépôt international:
**PCT/BE97/00078**

(87) Numéro de publication internationale:
**WO 98/001394 (15.01.1998 Gazette 1998/02)**

(54) **DISPOSITIF ET PROCEDE DE TRAITEMENT D'UN MILIEU LIQUIDE**

VORRICHTUNG UND PROZESS ZUR BEHANDLUNG EINES FLÜSSIGEN MEDIUMS

METHOD AND DEVICE FOR TREATING A LIQUID MEDIUM

(84) Etats contractants désignés:
**AT BE CH DE DK ES FI FR GB GR IE IT LI LU NL
PT SE**

(30) Priorité: **04.07.1996 BE 9600613**

(43) Date de publication de la demande:
**06.05.1999 Bulletin 1999/18**

(60) Demande divisionnaire:
**03075372.7 / 1 310 460**

(73) Titulaire: **ASHLAND INC.
Columbus, OH 43216 (US)**

(72) Inventeurs:
• **CORDEMANS DE MEULENAER, Eric
B-1970 Wezembeek-Oppem (BE)**
• **HANNECART, Baudouin
B-1180 Uccle (BE)**

• **CANIVET, Yves
B-1140 Evere (BE)**

(74) Mandataire: **Van Malderen, Joelle et al
Office Van Malderen,
Place Reine Fabiola 6/1
1083 Bruxelles (BE)**

(56) Documents cités:
**EP-A- 0 515 346          EP-A- 0 577 871
EP-A- 0 661 090          EP-A- 0 680 779
WO-A-80/00226          DE-A- 4 430 587
US-A- 2 717 874          US-A- 3 672 823
US-A- 4 003 832**

• **DMITRIEVA ET AL: "The Simultaneous Effect of
Two Acoustic Vibration Frequencies on the
Rates of Sonochemical Reactions" RUSSIAN
JOURNAL OF PHYSICAL CHEMISTRY, vol. 59,
no. 10, 1985, pages 2620-2623, XP002042376**

## Description

### Objet de l'invention

La présente invention concerne un nouveau dispositif et un procédé de traitement d'un milieu liquide destinés à éliminer des micro-organismes tels que des algues, des bactéries, des virus, etc., et/ou destinés à permettre une sursaturation en sels dans le milieu liquide.

### Arrière-plan technologique

Il est connu de l'homme de l'art que selon que l'on utilise des ultrasons de hautes fréquences ou de basses fréquences, on obtient sur des fluides des effets différents. Pour obtenir des effets mécaniques, les basses fréquences (généralement comprises entre 16 et 100 kHz) sont régulièrement utilisées.

Ces effets mécaniques qui sont dus à la cavitation, permettent par exemple de mélanger, d'émulsifier et de disperser des substances hétérogènes, de dégazer des liquides, etc. Ces effets mécaniques créent également de forts effets de cisaillement au sein des liquides, ce qui permet par exemple de perturber les cellules vivantes présentes dans ce milieu et provoquer jusqu'à la rupture des parois de ce type de cellules.

Ces mêmes effets mécaniques sont également utilisés pour nettoyer des surfaces par l'action des microjets de liquide produits par l'implosion des bulles de cavitation. De même, le fait de pouvoir mélanger très efficacement divers réactifs est utilisé de manière avantageuse pour favoriser les phénomènes de diffusion dans diverses réactions chimiques, surtout les réactions hétérogènes.

Si l'on utilise des hautes fréquences, généralement comprises entre 300 kHz et plusieurs MHz, les phénomènes de cavitation deviennent relativement moins énergétiques, et les effets mécaniques sont plus réduits que lorsque l'on utilisé des basses fréquences.

En appliquant des ultrasons de hautes fréquences à un milieu liquide, la durée de vie des bulles de cavitation produites est fortement réduite, et l'on assiste à des phénomènes chimiques en phase homogène qui se distinguent radicalement de ceux observés avec l'application des fréquences plus basses.

Un paramètre propre à l'utilisation d'ultrasons de hautes fréquences est que malgré la faible énergie mécanique nécessaire pour obtenir un dégazage des solutions, l'effet obtenu est plus élevé que lorsque l'on applique des ultrasons de basses fréquences qui, eux, nécessitent plus de puissance.

### Etat de la technique

Il est connu de différents documents de l'état de la technique que l'emploi l'ultrasons permet la détoxication de milieux liquides.

Le brevet américain US-5198122 décrit un procédé de détoxication de matériaux liquides et solides comprenant différents composés organiques toxiques, consistant en l'adjonction au milieu d'un catalyseur comprenant un borohydrate de sodium ou un hydrate de lithium aluminium aux dits matériaux, qui sont ensuite soumis aux ultrasons de manière à provoquer un phénomène de cavitation dans le mélange et à permettre la détoxication ou l'extraction plus aisée des substances contaminantes.

La demande de brevet allemand DE-4407564 décrit un orocédé d'oxydation de substances organiques ou de micro-organismes obtenu par addition d'oxydants (ozone) ou d'agents catalyseur de transfert de phase.

La demande de brevet japonais JP-930343777 (Marsima Aqua Syst. Corp.) décrit un procédé et une installation de purification d'un réservoir d'eau contenant du plancton végétal. Dans le premier réservoir de cette installation, on inactive le plancton végétal au moyen d'un vibrateur à ultrasons provoquant des phénomènes de cavitation dans l'eau. Le produit de ce traitement est ensuite transféré dans un second réservoir, où l'on favorise la prédation du plancton animal sur le plancton végétal inactivé.

De même, il a été proposé dans différents documents (JP-900401407 (Ina Shokuhin Kogyo KK) , JP-920035473 (Kubota Corp.), JP-920035472 (Kubota Corp.) et JP-920035896 (Kubota Corp.) de provoquer l'élimination de substances organiques chlorées ou d'éliminer des micro-organismes par un phénomène de cavitation au moyen d'ultrasons et d'injections d'ozone, de peroxydes et/ou de catalyseurs.

La demande de brevet japonais JP-820010627 (Hitachi Kiden Kogyo KK) décrit un procédé d'élimination de spores d'algues par un effet mécanique provoqué par l'utilisation d'ultrasons de hautes et basses fréquences, induisant la stérilisation et l'élimination des spores venant se fixer sur des plaques, et qui seront ensuite éliminés par l'effet purificateur de la cavitation des ultrasons de basses fréquences.

Tous ces exemples précédents utilisent les effets mécaniques des ultrasons de basses fréquences.

Il est connu par la publication scientifique de Pétrier C. et al. (Journal of Physical Chemistry, No. 98 - 10514-10520 (1994)) que la dégradation sonochimique des phénols dans des solutions aqueuses varie en fonction de la fréquence d'émission des ultrasons. Selon ce document, le taux de dégradation sonochimique serait directement lié à la présence dans la solution de radicaux libres comme par exemple H·, ·OH et HOO·, provenant de la sonolyse de l'eau sous ultrasons. Selon ce document, la production de ces radicaux libres, en particulier les radicaux ·OH et ·OOH, serait plus importante en travaillant avec l'émission d'ultrasons de hautes fréquences.

Comme il apparaît dans les illustrations de ce document, les radicaux produits par ultrasons induisent une fluorescence dont la distribution varie en fonction de la conception et du dessin de l'appareillage ainsi que de la fréquence d'émission des ultrasons. Cette fluorescence induite par les radicaux produits sous ultrasons est mise en évidence par l'adjonction de luminol à la composition aqueuse.

Cependant, ce document ne suggère nullement que l'on pourrait utiliser le phénomène d'oxydation obtenu par des ultrasons de hautes fréquences pour attaquer des micro-organismes présents dans ce milieu aqueux et obtenir ainsi leur élimination.

Le brevet américain US-2717874 décrit un dispositif de traitement d'un milieu aqueux comprenant des micro-organismes, par l'adjonction de réactifs chlorés. Ledit dispositif comprend une cloche (cellule) communiquant avec un réservoir du milieu aqueux à traiter, et comporte un émetteur d'ultrasons de hautes fréquences situé à la base de la cloche. En outre, la cloche présente un dispositif émetteur de bulles d'air au niveau du cône du geyser.

La demande de brevet WO93/13674 décrit un procédé de pasteurisation de lait ou de produits lactés de manière à éliminer un certain nombre de micro-organismes tels que des bactéries, des virus, des spores, etc. Le dispositif et le procédé décrits dans ce document sont basés sur l'utilisation de la cavitation obtenue à basses fréquences et à haute puissance pendant des temps d'application particulièrement longs.

La demande de brevet européen EP-A1-0633049 décrit un procédé de traitement d'un liquide par l'émission d'un champ d'ultrasons de manière à séparer les particules présentes dans ce liquide. Ce procédé utilise des hautes fréquences, qui permettent la séparation des microparticules par des champs d'ondes stationnaires dont on modifie la position par une variation de phase ou de fréquence, qui entraîne les particules qui restent ainsi concentrées dans les zones de ventres ou de noeuds. Dans le cas de particules biologiques, ce dispositif permet de créer un bioréacteur où les particules solides de toute nature restent confinées dans le champ ultrasonore, ce qui permet une filtration du milieu liquide. Il est à signaler que le dispositif permet d'obtenir la stérilisation de matériaux biologiques et l'inactivation de micro-organismes.

Le brevet américain US-4961860 décrit un procédé de traitement de l'eau, de manière à éliminer les micro-organismes présents par l'émission d'ultrasons de puissance à basses fréquences. Cependant, ce dispositif requiert une consommation énergétique particulièrement importante, de manière à obtenir un effet mécanique éliminant les micro-organismes.

## Buts de l'invention

La présente invention vise à fournir un dispositif et un procédé susceptibles de neutraliser, d'empêcher le développement et/ou d'éliminer de manière simple et peu coûteuse un certain nombre de cellules animales non différenciées telles que des cellules tumorales et/ou des micro-organismes d'un milieu liquide, en particulier des algues, des bactéries, des virus, etc., et qui ne présenteraient pas les inconvénients de l'état de la technique.

Un but particulier de la présente invention est de neutraliser, d'empêcher le développement et/ou d'éliminer lesdites cellules non différenciées ou micro-organismes, en particulier des algues, présents dans des milieux liquides tels que les eaux de piscine, les châteaux d'eau, les viviers, les aquariums ou tout autre réservoir d'eau, et par extension tout circuit industriel, par exemple un circuit de refroidissement, dont la qualité de l'eau est particulièrement importante, ou des liquides physiologiques tels que du sang ou du plasma, pouvant être extraits et/ou administrés à l'homme ou l'animal.

L'invention vise également à õbtenlr un dispositif en un procédé qui ne nécessitent pas l'adjonction de produits chimiques additionnels nécessaires pour obtenir les effets susmentionnés dans le milieu traité.

Un autre but de l'invention est d'obtenir un dispositif et un procédé ne consommant pas beaucoup d'énergie.

Un but complémentaire de la présente invention est d'obtenir un dispositif et un procédé permettant de traiter un milieu liquide avec des ultrasons n'engendrant pas de phénomène stationnaire.

Un dernier but de la présente invention est de fournir un dispositif et un procédé de traitement d'un milieu aqueux, permettant de provoquer une sursaturation en sels du milieu liquide, c'est-à-dire permettant d'obtenir des concentrations en sels supérieures à la solubilité naturelle de ces sels dans le milieu aqueux.

## Eléments caractéristiques de l'invention

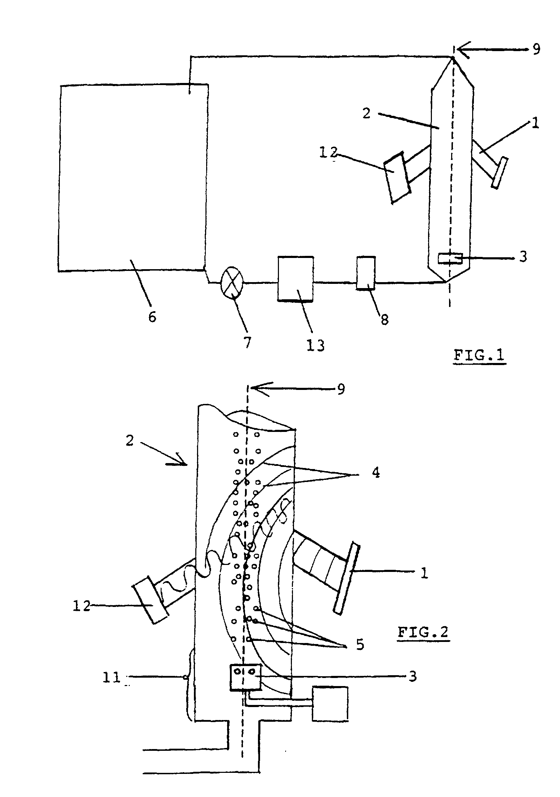

La présente invention telle que représentée dans les figures 1 et 2 concerne un dispositif ou installation de traitement d'un milieu liquide, de préférence aqueux (contenant éventuellement des cellules non différenciées ou des micro-organismes) , caractérisé en ce qu'il comprend, communiquant de préférence avec un "réservoir" 6 du milieu liquide à traiter, un compartiment 2, de préférence de forme cylindrique ou à section carrée, ledit compartiment 2 comportant (le long de sa paroi) un ou plusieurs émetteurs 1 d'ultrasons de hautes fréquences 4 émis vers l'intérieur du compartiment 2 (de préférence vers le centre de ce compartiment 2), et un ou plusieurs émetteurs 3 de microbulles de gaz 5 disposés de manière à assurer l'émission des microbulles de gaz 5 dans le champ des ultrasons 4 émis dans le compartiment 2.

On entend par "microbulles", des bulles de gaz ayant un diamètre moyen inférieur à 1 mm, de préférence inférieur ou égal à 50 $\mu$m, plus particulièrement inférieur à 30 $\mu$m.

On entend par "cellules non différenciées", des cellules présentant un effet hyperprolifératif telles que des cellules tumorales, des cellules de moelle osseuse ou des cellules totipotentes, notamment des cellules d'algues, etc.

On entend par "micro-organismes" présents dans un milieu liquide que l'on désire traiter, tous micro-organismes pathogènes ou non pathogènes susceptibles de provoquer des désagréments à l'homme, l'animal ou aux installations de transport et de stockage de milieux liquides. De tels micro-organismes peuvent être des bactéries, des virus ou des algues. La définition de ces micro-organismes comprend des êtres uni- et pluricellulaires, en particulier des algues, pouvant être présents dans différents milieux liquides, de préférence aqueux tels que des piscines, des réservoirs d'eau, des aquariums, des circuits industriels de refroidissement telles que des tours de refroidissement, etc.

On entend également par "milieu liquide " des liquides physiologiques pouvant être administrés à l'homme ou l'animal, ou extraits de l'homme ou de l'animal et réinjectés à celui-ci après traitement (traitement ex vivo de liquides physiologiques tels que le sang, le sérum, le liquide céphalo-rachidien, etc.), comme décrit dans le brevet américain US-5401237.

On entend par "hautes fréquences", des fréquences comprises entre 100 kHz et plusieurs MHz. De préférence, les hautes fréquences utilisées sont comprises entre 200 kHz et 10 MHz.

De préférence, ledit émetteur 3 de microbulles de gaz 5 est disposé à la base 11 du compartiment 2, c'est-à-dire dans la partie inférieure du compartiment 2, de manière à ce que les microbulles se déplacent par remontée naturelle ou entraînement du gaz dans le flux de liquide.

Avantageusement, les microbulles de gaz sont des microbulles d'air, d'ozone ou d'oxygène.

Selon l'invention, lesdits micro-organismes semblent être éliminés par la production dans leur environnement de radicaux de type H·, ·OH et HOO· susceptibles également de former de l'$H_2O_2$, cette molécule et/ou ces radicaux étant toxiques pour ces micro-organismes et provoquant ainsi leur inactivation et/ou leur destruction.

Les espèces créées proviendraient des réactions des ultrasons de hautes fréquences sur la molécule d'eau qui pourraient provoquer (en particulier en présence d'oxygène) les réactions suivantes :

$$H_2O \rightarrow H· + ·OH$$

$$H· + O_2 \rightarrow HOO·$$

$$HOO· + HOO· \rightarrow H_2O_2 + O_2$$

$$·OH + ·OH \rightarrow H_2O_2$$

De manière avantageuse, l'énergie nécessaire pour produire les espèces toxiques est réduite si l'on travaille en présence de ces microbulles.

En effet, il semblerait que l'injection de microbulles dans le champ des ultrasons provoque une augmentation du phénomène de sonoluminescence, par la superposition des microbulles aux bulles de cavitation provoquées par les ultrasons, ce qui semble multiplier le nombre d'espèces excitées et toxiques.

Ce phénomène est massivement observé lorsque l'on combine de manière synergique le traitement des ultrasons en présence des microbulles d'une taille adéquate.

Ce phénomène est accentué par la fragmentation des microbulles 5 créées lorsqu'elles passent dans le champ des ultrasons 4, en particulier lorsque le dispositif comporte plusieurs émetteurs successifs le long de la paroi du compartiment 2, qui assurent une plus grande fragmentation des microbulles et ce, de manière progressive.

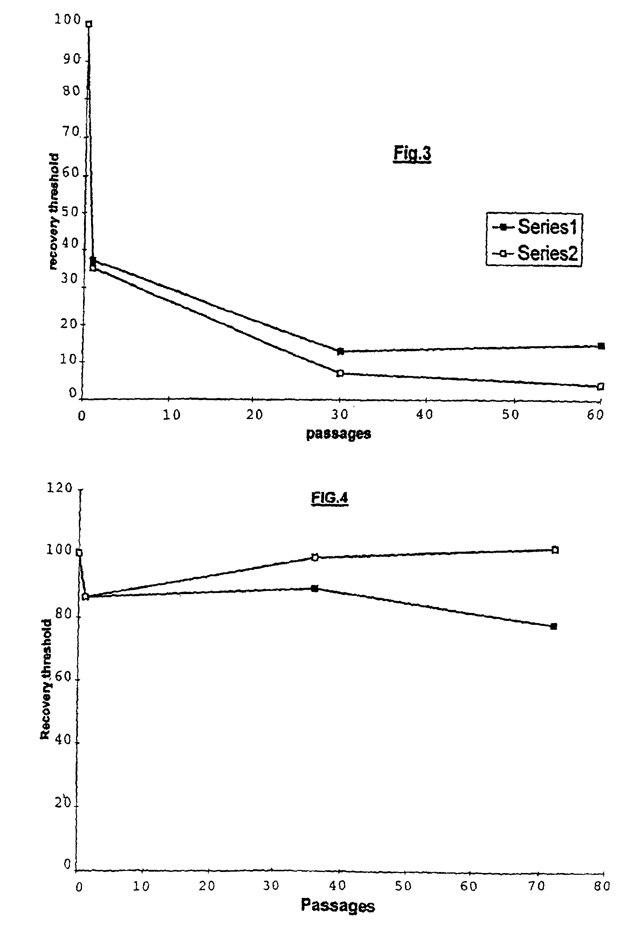

Ces caractéristiques sont notamment illustrées par les tests comparatifs des figures 3 à 5 annexées.

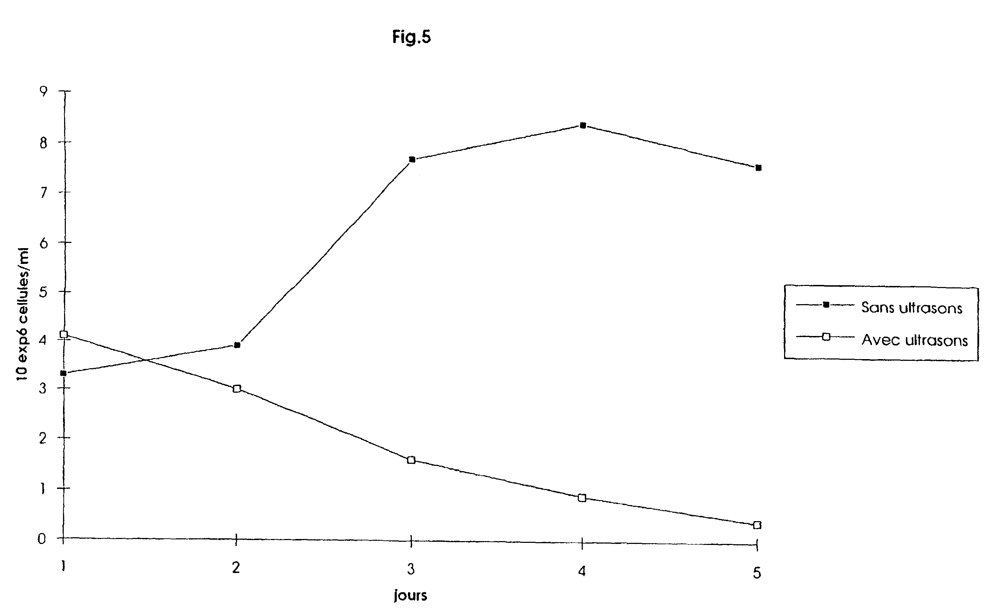

Les figures 3 et 4 reprennent un comptage des algues de type selenastrum capricornutum. On observe que ces algues placées dans des conditions optimales de culture voient leur potentiel de récupération (recovery threshold) inhibé lorsqu'elles sont traitées par le dispositif de l'invention (avec injection de microbulles (figure 3) et sans injection de microbulles (figure 4)). Pour une puissance ultrasonore donnée, l'efficacité du traitement est proportionnelle au temps de séjour dans le compartiment, et par conséquent, inversement proportionnelle au débit.

La figure 5 démontre, par rapport à un blanc de référence (traité dans toutes conditions de concentration, débit, température et bullage égales mais sans ultrasons), l'effet des ultrasons. On observe qu'une injection de microbulles mais sans traitement par ultrasons ne provoque pas d'inhibition de la croissance ni de diminution du nombre d'algues dans le milieu liquide.

En outre, le dispositif présente l'avantage qu'il n'y a pas lieu de dédicacer les ultrasons dans des zones particulières, car on constate que le système de traitement fonctionne par diffusion des produits formés in situ (par exemple radicaux et $H_2O_2$ formés) vers le réservoir 6 de milieu aqueux à traiter.

En outre, l'émetteur 1 d'ultrasons 4 du dispositif de l'invention est orienté de manière à ne pas provoquer de phénomènes d'ondes stationnaires, c'est-à-dire qu'il est orienté en oblique par rapport à l'axe 9 du compartiment 2 (angle aigu non perpendiculaire par rapport à cet axe 9) et par rapport au flux de liquide et au débit de microbulles 5 (voir figure 2). Cette caractéristique permet que toutes les microbulles 5 du compartiment 2 soient traitées de manière statistiquement identique, sans créer de zones stationnaires dans ledit compartiment 2.

Selon une forme d'exécution préférée de l'invention, le dispositif comprend également un émetteur de lumière 12 vers l'intérieur du compartiment 2 dans le champ des ultrasons 4, c'est-à-dire un émetteur de rayonnement électromagnétique dont la majorité de la fréquence est dans le visible. Cependant, pour certaines applications, de manière à éliminer certains micro-organismes particuliers, il peut être avantageux d'émettre également un rayonnement électromagnétique dont certaines fréquences correspondent au rayonnement ultraviolet (de type UVA, UVB ou UVC) ou d'autres types d'émissions énergétiques tels que infrarouge, laser, micro-ondes.

En effet, les Inventeurs ont observé de manière inattendue qu'un traitement comprenant l'émission de microbulles dans les champs combinés des ultrasons et du rayonnement lumineux, était particulièrement efficace pour obtenir une inactivation et une élimination des micro-organismes, en particulier des algues, présents dans le milieu liquide. Le phénomène de sonoluminescence peut favoriser la production d'espèces oxygénées extrêmement actives telles que le radical superoxyde ou l'oxygène de type singulet, qui sont responsables de toute une série de réactions biochimiques, extrêmement toxiques pour certains micro-organismes.

Il est connu qu'un tel phénomène peut être produit en présence de molécules dites photosensibilisatrices de manière à provoquer une action anti-tumorale sur certaines cellules cancéreuses (Umemura S.I., Japanese J. of Cancer Res., 81, pp. 962-966 (1990)). De telles molécules sont notamment des porphyrines, des chlorines, des tétracyclines, du bleu de méthylène, de la fluorescéine, de l'acridine, de la rhodamine, etc. Ces agents actifs sont injectés dans l'organisme ou administrés par voie orale pour être ensuite activés par sonoluminescence et produire ainsi l'oxygène de type singulet qui à son tour joue un rôle capital, notamment dans les processus biochimiques dus au stress oxydatif. L'action de l'oxygène singulet est d'oxyder les divers composants des cellules que sont les protéines, les lipides, les acides aminés et les nucléotides.

Selon la présente invention, il n'est pas toujours nécessaire d'ajouter dans le milieu à traiter un agent photosensibilisateur. En effet, les Inventeurs ont observé de manière inattendue que cet effet pouvait être produit in situ sur certains micro-organismes, notamment des algues, ou certaines cellules, en particulier des cellules non différenciées telles que des cellules tumorales présentes dans des liquides physiologiques tels que le sang, contenant déjà ces molécules photosensibilisatrices.

Selon une forme d'exécution préférée de l'invention, telle que représentée à la figure 1, le dispositif comprend, placé entre le réservoir 6 et le compartiment 2, un moyen de traitement électromagnétique 13 du milieu aqueux. Ce moyen 13 est donc séparé du compartiment 2 et placé en amont de celui-ci par rapport au flux du milieu aqueux à traiter.

On entend par "moyen de traitement électromagnétique d'un milieu aqueux", tout appareillage permettant de modifier, voire orienter, la formation de cristaux (modification de la morphologie des cristaux).

Un tel appareillage est notamment décrit dans la demande de brevet européenEP-0515346 qui décrit un générateur permettant de contrôler la nucléation et/ou la croissance de cristaux et/ou leur morphologie. Ce document décrit un exemple particulier permettant d'orienter les cristaux de carbonate de calcium vers une forme dendritique, ces cristaux étant dans ce cas dans une forme inapte à former des incrustations de tartre (dans les canalisations, échangeurs, etc.).

Un tel moyen de traitement électromagnétique du milieu aqueux permet, lorsque l'on soumet ledit milieu aqueux à l'action d'un champ créé entre au moins deux moyens de transfert (de manière à modifier la nucléation et/ou la formation et/ou la croissance de cristaux, en particulier de carbonate de calcium), au moins un desdits moyens de transfert recevant des signaux électriques sensiblement rectangulaires comprenant une première phase dans laquelle le signal est voisin d'un niveau inférieur et une deuxième phase dans laquelle le signal est voisin d'un niveau supérieur, lesdits signaux présentant, lorsqu'ils sont voisins du niveau inférieur ou du niveau supérieur, une série de variations amorties.

De façon avantageuse, les signaux correspondent à la superposition d'une onde rectangulaire de fréquence inférieure à 100000 Hz, de préférence comprise entre 1000 et 30000 Hz, et d'une onde sinusoïdale amortie de fréquence inférieure à 100000 Hz, de préférence comprise entre 1 MHz et 15 MHz, et l'amortissement de l'onde sinusoïdale est telle qu'après 5 périodes de ladite onde, l'amplitude de la variation est inférieure à 10% de celle de la variation initiale.

Dans une forme d'exécution particulière de l'invention, pour chaque phase de l'onde rectangulaire, cette dernière est associée à une seule onde sinusoïdale amortie, la fréquence de l'onde amortie étant supérieure à celle de l'onde rectangulaire et l'onde amortie étant émise pour chaque phase de l'onde rectangulaire, le temps de vie de l'onde amortie étant compris entre 10 ns et 500 µs, de préférence entre 100 ns ET 100 µs.

Dans un exemple de réalisation particulier, la différence de potentiel existant entre le niveau inférieur et le niveau supérieur de l'onde rectangulaire est comprise entre 5 et 200 V, de préférence entre 12 et 60 V, tandis que les ondes amorties sont des ondes dont les variations sont situées dans une bande s'étendant entre un niveau minimum et un niveau maximum, la différence de potentiel entre lesdits niveaux étant comprise entre 1 et 60 V, de préférence entre 10 et 35 V.

Ledit moyen de traitement électromagnétique d'un milieu aqueux peut être également l'appareillage tel que décrit dans le brevet américain US-4879045.

Les Inventeurs ont découvert de manière inattendue que le dispositif de l'invention, comprenant un moyen assurant un traitement électromagnétique du milieu liquide disposé en amont des éléments assurant un traitement du milieu aqueux par microbullage dans un champ d'ultrasons de hautes fréquences, permet d'obtenir une large plage de sursaturation en sels du milieu liquide avant que le phénomène de cristallisation n'apparaisse.

On entend par "sursaturation en sels", la possibilité d'augmenter la concentration en sels du milieu aqueux (au-delà de la concentration correspondant au produit de solubilité des sels) sans obtenir une précipitation desdits sels.

Par exemple, lesdits sels sont constitués par du carbonate de calcium présent dans le milieu aqueux, qui peut être apporté via un raccordement du milieu aqueux à de l'eau du réseau de captage et de distribution ou par ajout desdits sels dans le milieu liquide.

Cette quantité importante de sels présente dans le milieu aqueux peut également être obtenue par une évaporation partielle de l'eau, ce phénomène étant particulièrement important dans les tours de refroidissement.

Le milieu liquide peut également comprendre une sursaturation en d'autres sels, tels que des phosphates ou des sulfates de métaux alcalins ou alcalinoterreux, etc.

De manière inattendue, les Inventeurs ont observé qu'il est possible d'obtenir une concentration en sels, en particulier en carbonate de calcium, dans le milieu liquide traité, à des concentrations 10 fois supérieures à la saturation (dissolution maximale avant précipitation).

De manière avantageuse, on observe également que l'eau ainsi traitée reste limpide.

Un tel dispositif permet de développer des systèmes anti-tartre, de produire des eaux sursaturées en sels, de provoquer des effets biotoxiques sur certains micro-organismes dus à la présence d'eau sursaturée en certains sels, etc.

Un tel dispositif permet également d'obtenir des milieux concentrés sans précipitation et créer des réactions chimiques particulières par adjonction de réactifs.

De manière inattendue, les Inventeurs ont découvert que pour obtenir de manière optimale ce phénomène de sursaturation en sels des milieux aqueux traités, il est nécessaire que le dispositif de traitement électromagnétique soit séparé du compartiment où se fait l'émission de microbulles dans le champ d'ultrasons, et de préférence qu'il la précède.

Le dispositif selon la présente invention peut comprendre, disposés entre le réservoir 6 contenant le milieu liquide à traiter et le tube 2, une pompe 7 assurant la circulation du milieu liquide, ainsi que des moyens de récupération, de préférence par filtration, centrifugation ou précipitation (tels que des cyclones, etc.), des micro-organismes et/ou des sels présents dans le milieu liquide.

Un autre aspect de la présente invention concerne l'utilisation du dispositif selon l'invention pour neutraliser et/ou éliminer les micro-organismes, en particulier des algues, présents dans un milieu liquide à traiter.

On entend par "neutralisation et/ou élimination d'un micro-organisme", la réduction ou le maintien en-dessous d'un niveau de concentration (variant pour chaque micro-organisme et chaque milieu liquide) des micro-organismes susceptibles de se multiplier dans ce milieu liquide.

Un dernier aspect de la présente invention concerne l'utilisation du dispositif selon l'invention pour provoquer une sursaturation en sels du milieu liquide, de préférence du milieu aqueux, c'est-à-dire obtenir des concentrations en sels supérieures à la solubilité naturelle de ces sels dans le milieu liquide, de préférence une concentration 5 fois, voire 10 fois, supérieure à la solubilité naturelle de ces sels.

Les milieux sursaturés ainsi obtenus permettent leur utilisation pour provoquer des réactions chimiques particulières par adjonction de réactifs, conférant aux produits formés des caractéristiques originales (taille, forme, surface spécifique, etc.). Les réactifs ajoutés peuvent être solides, liquides, gazeux ou constitués de mélanges hétérogènes.

## Revendications

1. Dispositif de traitement d'un milieu liquide comprenant, communiquant avec un réservoir (6) du milieu liquide à traiter, un compartiment (2) comportant un émetteur (1) d'ultrasons (4) émis vers l'intérieur du compartiment (2) et un émetteur (3) de microbulles de gaz (5) présentant un diamètre moyen inférieur à 1 mm, **caractérisé en ce que** ledit émetteur (1) d'ultrasons (4) est un émetteur (1) d'ultrasons (4) de hautes fréquences comprises entre 100 KHz et plusieurs MHz **en ce que** l'émetteur (3) de microbulles de gaz (5) est disposé de manière à assurer l'émission des microbulles de gaz (5) dans le champ des ultrasons (4) de hautes fréquences émis dans le compartiment (2) .

2. Dispositif selon la revendication 1, **caractérisé en ce que** les microbulles de gaz (5) sont des microbulles d'air ou d'oxygène.

3. Dispositif selon la revendication 1 ou 2, **caractérisé en ce que** le milieu liquide est un milieu liquide aqueux.

4. Dispositif selon la revendication 3, **caractérisé en ce que** le milieu liquide aqueux est choisi parmi le groupe constitué par les piscines, les réservoirs d'eau, les aquariums ou les circuits industriels de refroidissement.

**5.** Dispositif selon la revendication 4, **caractérisé en ce que** le circuit industriel de refroidissement est une tour de refroidissement.

**6.** Dispositif selon la revendication 3, **caractérisé en ce que** le milieu aqueux est un liquide physiologique pouvant être administré à l'homme ou à l'animal ou extrait de l'homme ou de l'animal.

**7.** Dispositif selon la revendication 6, **caractérisé en ce que** le liquide physiologique est choisi parmi le groupe constitué par le sang, le sérum ou le liquide céphalo-rachidien.

**8.** Dispositif selon l'une quelconque des revendications précédentes, **caractérisé en ce que** le diamètre moyen des microbulles de gaz (5) est inférieur à 50 μm.

**9.** Dispositif selon l'une quelconque des revendications précédentes, **caractérisé en ce que** le diamètre moyen des microbulles de gaz (5) est inférieur à 30 μm.

**10.** Dispositif selon l'une quelconque des revendications précédentes, **caractérisé en ce que** l'émetteur (3) de microbulles de gaz (5) est disposé à la base (11) du compartiment (2) et **en ce que** les microbulles de gaz (5) se déplacent par remontée naturelle ou entraînement des microbulles de gaz (5) dans le milieu aqueux à traiter.

**11.** Dispositif selon l'une quelconque des revendications précédentes, **caractérisé en ce que** le compartiment (2) présente une forme cylindrique ou une forme à section carrée et dans lequel l'émetteur (1) d'ultrasons (4) est orienté en oblique par rapport à l'axe (9) dudit compartiment (2).

**12.** Dispositif selon l'une quelconque des revendications précédentes, **caractérisé en ce qu'**il comporte plusieurs émetteurs (1) d'ultrasons (4) successifs le long de la paroi du compartiment (2).

**13.** Dispositif selon l'une quelconque des revendications précédentes, **caractérisé en ce que** le compartiment (2) comporte également un émetteur (12) de lumière émise dans le champ des ultrasons (4) et dont le rayonnement électromagnétique est majoritairement dans le visible.

**14.** Dispositif selon l'une quelconque des revendications précédentes, **caractérisé en ce qu'**il comporte un émetteur de rayonnement électromagnétique.

**15.** Dispositif selon la revendication 14, **caractérisé en ce qu'**il comporte un émetteur de rayonnement électromagnétique dont certaines fréquences correspondent au rayonnement ultraviolet (UVA, UVB, UVC) ou un autre type d'émission énergétique tel que l'infrarouge, le rayonnement laser, les micro-ondes.

**16.** Dispositif selon l'une quelconque des revendications précédentes, **caractérisé en ce qu'**il comporte, placé entre le réservoir (6) et le compartiment (2), un moyen de traitement électromagnétique (13) du milieu liquide de manière à modifier la nucléation, la formation et/ou la croissance de cristaux de sels.

**17.** Dispositif selon l'une quelconque des revendications précédentes, **caractérisé en ce qu'**il comporte un ou plusieurs moyens permettant de recueillir les micro-organismes et/ou les sels présents dans le milieu liquide traité.

**18.** Dispositif selon la revendication 17, **caractérisé en ce que** les moyens permettant de recueillir les micro-organismes et/ou les sels présents dans le milieu liquide traité sont un filtre ou un cyclone.

**19.** Utilisation du dispositif selon l'une quelconque des revendications précédentes, pour neutraliser, éliminer et/ou empêcher le développement de cellules non différenciées et/ou de micro-organismes présents dans un milieu liquide.

**20.** Utilisation du dispositif selon la revendication 19 **caractérisée en ce que** le micro-organisme est un micro-organisme pathogène ou non pathogène.

**21.** Utilisation selon la revendication 19 ou 20, **caractérisée en ce que** les micro-organismes sont choisis parmi le groupe constitué par les bactéries, les virus et/ou les algues.

**22.** Utilisation selon la revendication 19, **caractérisée en ce que** les cellules non différenciées sont des cellules présentant un effet hyperprolifératif.

**23.** Utilisation selon la revendication 22, **caractérisée en ce que** les cellules présentant un effet hyperprolifératif sont choisies parmi le groupe constitué par les cellules cancéreuses, les cellules de la moelle osseuse ou les cellules totipotentes.

**24.** Utilisation du dispositif selon la revendication 16, pour provoquer une sursaturation en sels du milieu liquide.

**25.** Utilisation selon la revendication 24, **caractérisée en ce que** la sursaturation en sels du milieu aqueux est une concentration en sels supérieure à la solubilité naturelle de ces sels dans le milieu liquide.

**26.** Utilisation selon la revendication 25, **caractérisée en ce que** la concentration en sels est 5 fois supérieure à la solubilité naturelle de ces sels dans le milieu liquide.

**27.** Utilisation du dispositif selon l'une quelconque des revendications 1 à 18, pour provoquer par adjonction de réactifs au milieu liquide traité des réactions chimiques, des réactions de précipitation ou des réactions de cristallisation.

**28.** Procédé pour neutraliser, éliminer et/ou empêcher le développement de cellules non-différenciées et/ou de micro-organismes présents dans un milieu liquide et/ou pour obtenir une sursaturation en sels dans un milieu liquide dans lequel des microbulles de gaz (5) présentant un diamètre moyen inférieur à 1 mm et des ultrasons (4) sont émis à l'intérieur d'un compartiment (2) communiquant avec un réservoir (6) du milieu liquide à traiter et dans lequel lesdites microbulles de gaz (5) sont émises dans le champ d'ultrasons (4) émis dans le compartiment (2) **caractérisé en ce que** lesdits ultrasons (4) sont des ultrasons de hautes fréquences comprises entre 100 KHz et plusieurs MHz.

**29.** Procédé selon la revendication 28, **caractérisé en ce que** le milieu liquide est un milieu liquide aqueux.

**30.** Procédé selon l'une quelconque des revendications précédentes 28 à 29, **caractérisé en ce que** les microbulles de gaz (5) sont des microbulles d'air ou d'oxygène.

**31.** Procédé selon la revendication 29, **caractérisé en ce que** le milieu liquide aqueux est choisi parmi le groupe constitué par les piscines, les réservoirs d'eau, les aquariums ou les circuits industriels de refroidissement.

**32.** Procédé selon la revendication 31, **caractérisé en ce que** le circuit industriel de refroidissement est une tour de refroidissement.

**33.** Procédé selon la revendication 29, **caractérisé en ce que** le milieu liquide aqueux est un liquide physiologique pouvant être administré à l'homme ou à l'animal ou extrait de l'homme ou de l'animal.

**34.** Procédé selon la revendication 33, **caractérisé en ce que** le liquide physiologique est choisi parmi le groupe constitué par le sang, le sérum ou le liquide céphalo-rachidien.

**35.** Procédé selon l'une quelconque des revendications précédentes 28 à 34, **caractérisé en ce que** le diamètre moyen des microbulles de gaz (5) est inférieur à 50 µm.

**36.** Procédé selon l'une quelconque des revendications précédentes 28 à 35, **caractérisé en ce que** le diamètre moyen des microbulles de gaz (5) est inférieur à 30 µm.

**37.** Procédé selon l'une quelconque des revendications précédentes 28 à 36, **caractérisé en ce que** les ultrasons (4) émis dans le compartiment (2) n'engendrent pas de phénomène de champ stationnaire.

**38.** Procédé selon l'une quelconque des revendications précédentes 28 à 37, **caractérisé en ce que** de la lumière dont le rayonnement électromagnétique est majoritairement dans le visible, est émise dans le champ des ultrasons (4).

**39.** Procédé selon l'une quelconque des revendications précédentes 28 à 38, **caractérisé en ce que** le milieu liquide aqueux est soumis à un traitement électromagnétique de manière à modifier la nucléation, la formation et/ou la croissance de cristaux de sels dans ledit milieu liquide aqueux.

**40.** Procédé selon l'une quelconque des revendications précédentes 28 à 38, **caractérisé en ce que** lesdits micro-organismes sont des micro-organismes pathogènes ou non pathogènes.

**41.** Procédé selon l'une quelconque des revendications précédentes 28 à 40, **caractérisé en ce que** lesdits micro-organismes sont choisis parmi le groupe constitué par les bactéries, les virus et/ou les algues.

**42.** Procédé selon l'une quelconque des revendications précédentes 28 à 39, **caractérisé en ce que** les cellules non différenciées sont des cellules présentant un effet hyperprolifératif.

**43.** Procédé selon la revendication 42, **caractérisé en ce que** les cellules présentant un effet hyperprolifératif sont choisies parmi le groupe constitué par les cellules cancéreuses, les cellules de la moelle osseuse ou les cellules totipotentes.

**44.** Procédé selon l'une quelconque des revendications précédentes 28 à 43, **caractérisé en ce que** la sursaturation en sels du milieu liquide aqueux est une concentration en sels supérieure à la solubilité naturelle de ces sels dans les milieux aqueux.

**45.** Procédé selon la revendication 44, **caractérisé en ce que** la concentration en sels est 5 fois supérieure à la solubilité naturelle de ces sels dans les milieux aqueux.

**Patentansprüche**

1. Vorrichtung zum Behandeln eines flüssigen Mediums, welches aufweist ; ein mit einem Behälter (6) für das zu behandelnde flüssige Medium in Verbindung stehendes Abteil (2) mit einem Sender (1) von Ultraschallwellen (4), die zum Innern des Abteils (2) ausgestrahlt werden, sowie einen Sender (3) von Gasmikroblasen (5), welche einen Durchmesser von weniger als 1 mm aufweisen, **dadurch gekennzeichnet, dass** der besagte Sender (1) von Ultraschallwellen (4) aus einem Sender (1) von Ultraschallwellen (4) mit zwischen 100 KHz und mehreren MHz liegenden Hochfrequenzen besteht, und dass der Sender (3) vonGasmikroblasen (5) auf eine solche Art und Weise angeordnet ist, dass die Emission der Gasmikroblasen (5) in das Feld der Ultraschallwellen (4) mit Hochfrequenzen, die in dem Abteil (2) ausgestrahlt werden, gewährleistet ist.

2. Vorrichtung gemäß Anspruch 1, **dadurch gekennzeichnet, dass** die Gasmikroblasen (5) solche Mikroblasen sind, die aus Luft oder aus Sauerstoff bestehen.

3. Vorrichtung gemäß Anspruch 1 oder 2, **dadurch gekennzeichnet, dass** das flüssige Medium aus einem flüssigen wässrigen Medium besteht.

4. Vorrichtung gemäß Anspruch 3, **dadurch gekennzeichnet, dass** das flüssige wässrige Medium aus der Gruppe bestehend aus Schwimmbecken, Wasserbehältern, Aquarien, oder industriellen Kühlkreisläufen ausgewählt wird.

5. Vorrichtung gemäß Anspruch 4, **dadurch gekennzeichnet, dass** der industrielle Kühlkreislauf aus einem Kühlturm besteht.

6. Vorrichtung gemäß Anspruch 3, **dadurch gekennzeichnet, dass** das wässrige Medium aus einer physiologischen Flüssigkeit besteht, welche dem Menschen oder dem Tier verabreicht werden kann oder welche aus dem Menschen oder aus dem Tier extrahiert werden kann.

7. Vorrichtung gemäß Anspruch 6, **dadurch gekennzeichnet, dass** die physiologische Flüssigkeit aus der Gruppe bestehend aus Blut, Serum oder Gehirn-Rückenmarks-Flüssigkeit ausgewählt wird.

8. Vorrichtung gemäß irgendeinem der vorhergehenden Ansprüche, **dadurch gekennzeichnet, dass** der mittlere Durchmesser der Gasmikroblasen (5) kleiner ist als 50 μm.

9. Vorrichtung gemäß irgendeinem der vorhergehenden Ansprüche, **dadurch gekennzeichnet, dass** der mittlere Durchmesser der Gasmikroblasen (5) kleiner ist also 30 μm.

10. Vorrichtung gemäß irgendeinem der vorhergehenden Ansprüche, **dadurch gekennzeichnet, dass** der Sender (3) von Gasmikroblasen (5) an der Basis (1) des Abteils (2) angeordnet ist und dass die Gasmikroblasen (5) sich durch ein natürliches Aufsteigen oder durch ein Mitreißen der Gasmikroblasen (5) in dem zu behandelnden wässrigen Medium bewegen.

11. Vorrichtung gemäß irgendeinem der vorhergehenden Ansprüche, **dadurch gekennzeichnet, dass** das Abteil (2) eine zylindrische Form aufweist oder eine Form mit einem quadratischen Querschnitt innerhalb dessen der Sender (1) von Ultraschallwellen (4) schräg ausgerichtet ist in Bezug auf die Achse (9) des besagten Abteils (2).

12. Vorrichtung gemäß irgendeinem der vorhergehenden Ansprüche, **dadurch gekennzeichnet, dass** dieselbe mehrere aufeinander folgende Sender (1) von Ultraschallwellen (4) entlang der Wand des Abteils (2) aufweist.

13. Vorrichtung gemäß irgendeinem der vorhergehenden Ansprüche, **dadurch gekennzeichnet, dass** das Abteil (2) ebenfalls einen Lichtsender (12) für das in dem Feld der Ultraschallwellen (4) ausgestrahlte Licht aufweist, wobei dessen elektromagnetische Strahlung zum größten Teil in dem sichtbaren Bereich liegt.

14. Vorrichtung gemäß irgendeinem der vorhergehenden Ansprüche, **dadurch gekennzeichnet, dass** dieselbe einen Sender von elektromagnetischer Strahlung aufweist.

15. Vorrichtung gemäß Anspruch 14, **dadurch gekennzeichnet, dass** dieselbe einen Sender von elektromagnetischen Strahlung aufweist, bei welchen gewisse Frequenzen der ultravioletten Strahlung (UVA, UVB, UVC) entsprechen oder einer anderen Art von energetischer Strahlung wie etwa dem Infrarot, der Laserstrahlung, den Mikrowellen.

16. Vorrichtung gemäß irgendeinem der vorhergehenden Ansprüche, **dadurch gekennzeichnet, dass** dieselbe, zwischen dem Behälter (6) und dem Abteil (2) angeordnet, ein Hilfsmittel zur elektromagnetischen Behandlung (13) des flüssigen Mediums umfasst, derart dass die Kernbildung, die Bildung und / oder das Wachsen der Salzkristalle verändert werden.

17. Vorrichtung gemäß irgendeinem der vorhergehenden Ansprüche, **dadurch gekennzeichnet, dass** dieselbe eines oder mehrere Hilfsmittel umfasst, welche es erlauben, die Mikroorganismen und / oder die Salze aufzunehmen, welche in dem behandelten flüssigen Medium vorhanden sind.

18. Vorrichtung gemäß Anspruch 17, **dadurch gekennzeichnet, dass** die Hilfsmittel, welche es erlauben die Mikroorganismen und / oder die Salze aufzunehmen, welche in dem behandelten flüssigen Medium vorhanden sind, aus einem Filter oder einem Zyklon bestehen.

19. Anwendung der Vorrichtung gemäß irgendeinem der vorhergehenden Ansprüche zum Neutralisieren, Entfernen und / oder Verhindern der Entwicklung von nicht differenzierten Zellen und / oder von Mikroorganismen, welche in dem flüssigen Medium vorhanden sind.

20. Anwendung der Vorrichtung gemäß Anspruch 19, **dadurch gekennzeichnet, dass** der Mikroorganismus aus einem pathogenen oder aus einem nicht pathogenen Mikroorganismus besteht.

21. Anwendung gemäß Anspruch 19 oder 20, **dadurch gekennzeichnet, dass** die Mikroorganismen aus der Gruppe bestehend aus den Bakterien, den Viren und / oder den Algen ausgewählt werden.

22. Anwendung gemäß Anspruch 19, **dadurch gekennzeichnet, dass** die nicht differenzierten Zellen solche Zellen sind, welche eine hyperproliferative Wirkung aufweisen.

23. Anwendung gemäß Anspruch 22, **dadurch gekennzeichnet, dass** die eine hyperproliferative Wirkung aufweisenden Zellen aus der Gruppe bestehend aus den Krebszellen, den Knochenmarkszellen oder den totipotenten Zellen ausgewählt werden.

24. Anwendung der Vorrichtung gemäß Anspruch 16 zum Hervorrufen einer Übersättigung an Salzen des flüssigen Mediums.

25. Anwendung gemäß Anspruch 24, **dadurch gekennzeichnet, dass** die Übersättigung an Salzen des flüssigen Mediums eine Konzentration an Salzen ist, welche höher liegt als die natürliche Löslichkeit dieser Salze in dem flüssigen Medium.

26. Anwendung gemäß Anspruch 25, **dadurch gekennzeichnet, dass** die Konzentration an Salzen fünfmal höher liegt als die natürliche Löslichkeit dieser Salze in dem flüssigen Medium.

27. Anwendung der Vorrichtung gemäß irgendeinem der Ansprüche 1 bis 18 zum Hervorrufen der chemischen Reaktionen, der Fällungsreaktionen oder der Kristallisationsreaktionen durch Hinzufügen von Reagenzien zu dem behandelten flüssigen Medium.

28. Verfahren zum Neutralisieren, Entfernen und / oder Verhindern der Entwicklung von nicht differenzierten Zellen und / oder von Mikroorganismen, welche in einem flüssigen Medium vorhanden sind, und / oder zum Hervorrufen einer Übersättigung an Salzen eines flüssigen Mediums in welchem Gasmikroblasen (5) emittiert werden, welche einen mittleren Durchmesser von weniger als 1 mm aufweisen, und Ultraschallwellen (4) im Innern eines Abteils (2) ausgestrahlt werden, welches in Verbindung mit einem Behälter (6) für das zu behandelnde flüssige Medium steht und in welchem die besagten Gasmikroblasen (5) in das Feld der Ultraschallwellen (4), die in dem Abteil (2) ausgestrahlt werden, emittiert werden, **dadurch gekennzeichnet, dass** die besagten Ultraschallwellen (4) solche Ultraschallwellen mit Hochfrequenzen sind, welche zwischen 100 KHz und mehreren MHz liegen.

29. Verfahren gemäß Anspruch 28, **dadurch gekennzeichnet, dass** das flüssige Medium aus einem flüssigen wässrigen Medium besteht.

30. Verfahren gemäß irgendeinem der vorhergehenden Ansprüche 28 bis 29, **dadurch gekennzeichnet, dass** die Gasmikroblasen (5) Mikroblasen aus Luft oder aus Sauerstoff sind.

31. Verfahren gemäß Anspruch 29, **dadurch gekennzeichnet, dass** das flüssige wässrige Medium aus der Gruppe bestehend aus Schwimmbecken, Wasserbehältern, Aquarien oder industriellen Kühlkreisläufen ausgewählt wird.

32. Verfahren gemäß Anspruch 31, **dadurch gekennzeichnet, dass** der industrielle Kühlkreislauf aus einem Kühlturm besteht.

33. Verfahren gemäß Anspruch 29, **dadurch gekennzeichnet, dass** das flüssige wässrige Medium aus einer physiologischen Flüssigkeit besteht, welche dem Menschen oder dem Tier verabreicht werden kann oder welche aus dem Menschen oder aus dem Tier extrahiert werden kann.

34. Verfahren gemäß Anspruch 33, **dadurch gekennzeichnet, dass** die physiologische Flüssigkeit aus der Gruppe bestehend aus Blut, Serum oder Gehirn-Rückenmarks-Flüssigkeit ausgewählt wird.

35. Vorrichtung gemäß irgendeinem der vorhergehenden Ansprüche 28 bis 34, **dadurch gekennzeich-**

**net; dass** der mittlere Durchmesser der Gasmikroblasen (5) kleiner ist als 50 μm.

36. Vorrichtung gemäß irgendeinem der vorhergehenden Ansprüche 28 bis 35, **dadurch gekennzeichnet, dass** der mittlere Durchmesser der Gasmikroblasen (5) kleiner ist als 30 μm.

37. Vorrichtung gemäß irgendeinem der vorhergehenden Ansprüche 28 bis 36, **dadurch gekennzeichnet, dass** die Ultraschallwellen (4), die in das Abteil (2) ausgestrahlt werden, kein Phänomen eines stationären Feldes hervorruft.

38. Vorrichtung gemäß irgendeinem der vorhergehenden Ansprüche 28 bis 37, **dadurch gekennzeichnet, dass** das Licht dessen elektromagnetische Strahlung zum größten Teil in dem sichtbaren Bereich liegt, in das Feld der Ultraschallwellen (4) ausgestrahlt wird.

39. Vorrichtung gemäß irgendeinem der vorhergehenden Ansprüche 28 bis 38, **dadurch gekennzeichnet, dass** das flüssige wässrige Medium einer elektromagnetischen Behandlung unterzogen wird, derart dass die Kernbildung, die Bildung und / oder das Wachsen der. Salzkristalle in dem besagten wässrigen flüssigen Medium verändert werden.

40. Vorrichtung gemäß irgendeinem der vorhergehenden Ansprüche 28 bis 38, **dadurch gekennzeichnet, dass** die besagten Mikroorganismen pathogene oder nicht pathogene Mikroorganismen sind.

41. Vorrichtung gemäß irgendeinem der vorhergehenden Ansprüche. 28 bis 40, **dadurch gekennzeichnet, dass** die besagten Mikroorganismen aus der Gruppe bestehend aus den Bakterien, den Viren und / oder den Algen ausgewählt werden.

42. Vorrichtung gemäß irgendeinem der vorhergehenden Ansprüche 28 bis 39, **dadurch gekennzeichnet, dass** die nicht diffferenzierten Zellen solche Zellen sind, welche eine hyperproliferative Wirkung aufweisen.

43. Vorrichtung gemäß Anspruch 42, **dadurch gekennzeichnet, dass** die eine hyperproliferative Wirkung aufweisenden Zellen aus der Gruppe bestehend aus den Krebszellen, den Knochenmarkszellen oder den totipotenten Zellen ausgewählt werden.

44. Vorrichtung gemäß irgendeinem der vorhergehenden Ansprüche 28 bis 43, **dadurch gekennzeichnet, dass** die Übersättigung an Salzen des flüssigen wässrigen Mediums eine Konzentration an Salzen ist, welche höher liegt als die natürliche Löslichkeit dieser Salze in den wässrigen Medien.

45. Vorrichtung gemäß Anspruch 44, **dadurch gekennzeichnet, dass** die Konzentration ah Salzen fünfmal höher liegt als die natürliche Löslichkeit dieser Salze in den wässrigen Medien.

**Claims**

1. Device for treating a liquid medium which comprises, communicating with a reservoir (6) of the liquid medium to be treated, a compartment (2) comprising an emitter (1) which emits ultrasound (4) into the compartment (2) and an emitter (3) of gas microbubbles (5) with an average diameter of less than 1 mm, **characterized in that** the said ultrasound (4) emitter (1) is a high frequency ultrasound (4) emitter (1) , said ultrasound having a frequency comprised between 100 KHz and several MHz and **in that** the gas microbubbles (5) emitter (3) is arranged so as to emit gas microbubbles (5) into the high frequency ultrasound (4) field emitted into the compartment (2).

2. Device according to claim 1, **characterized in that** the gas microbubbles (5) are air or oxygen microbubbles.

3. Device according to claim 1 or 2, **characterized in that** the liquid medium is an aqueous liquid medium.

4. Device according to claim 3, **characterized in that** the aqueous liquid medium is selected from the group consisting of swimming pools, water reservoirs, aquariums or industrial cooling circuits.

5. Device according to claim 4, **characterized in that** the industrial cooling circuit is a cooling tower.

6. Device according to claim 3, **characterized in that** the liquid medium is a physiological liquid which can be administered to man or animal or extracted from man or animal.

7. Device according to claim 6, **characterized in that** the physiological liquid is selected from the group consisting of blood, serum or cerebrospinal fluid.

8. Device according to any of the preceding claims, **characterized in that** the average diameter of the gas microbubbles (5) is less than 50 μm.

9. Device according to any of the preceding claims, **characterized in that** the average diameter of the gas microbubbles (5) is less than 30 μm.

**10.** Device according to any of the preceding claims, **characterized in that** the gas microbubbles (5) emitter (3) is placed at the base (11) of the compartment (2) and **in that** the gas microbubbles (5) move by rising naturally or by entrainment of the gas microbulles (5) in the aqueous medium to be treated.

**11.** Device according to any of the preceding claims, **characterized in that** the compartment (2) presents a cylindrical shape or a rectangular cross-section shape and wherein the ultrasound (4) emitter (1) is oriented obliquely relatively to the axis (9) of said compartment (2).

**12.** Device according to any of the preceding claims, **characterized in that** it comprises several successive ultrasound (4) emitters (1) along the wall of the compartment (2).

**13.** Device according to any of the preceding claims, **characterized in that** the compartment (2) comprises a light emitter (12) whose light is emitted into the ultrasound (4) field and whose electromagnetic radiation is mainly in the visible range.

**14.** Device according to any of the preceding claims, **characterized in that** it comprises an electromagnetic radiation emitter.

**15.** Device according to claim 14, **characterized in that** it comprises an electromagnetic radiation emitter whose frequencies correspond to ultraviolet radiation (UVA, UVB, UVC) or another type of energy emissions such as infrared, laser radiation, microwaves.

**16.** Device according to any of the preceding claims, **characterized in that** it comprises, placed between the reservoir (6) and the compartment (2), a means for electromagnetic treatment (13) of the liquid medium so as to modify nucleation, formation and/or growth of salt crystals.

**17.** Device according to any of the preceding claims, **characterized in that** it comprises one or several means for recovering the microorganisms and/or the salts present in the treated liquid medium.

**18.** Device according to claim 17, **characterized in that** the means for recovering the microorganisms and/or the salts present in the treated liquid medium are a filter or a cyclone.

**19.** Use of the device according to any of the preceding claims, to neutralize, to remove and/or to prevent the growth of undifferentiated cells and/or microorganisms present in a liquid medium.

**20.** Use of the device according to claim 19, wherein said microorganism is a pathogenic or non-pathogenic microorganism.

**21.** Use according to claim 19 or 20, **characterized in that** the microorganisms are selected from the group consisting of bacteria, viruses and/or algae.

**22.** Use according to claim 19, **characterized in that** the undifferentiated cells are cells presenting an hyperproliferative effect.

**23.** Use according to claim 22, **characterized in that** the cells presenting an hyperproliferative effect are selected from the group consisting of cancer cells, bone marrow cells or totipotent cells.

**24.** Use of the device according to claim 16, to bring about a supersaturation with salts in the liquid medium.

**25.** Use according to claim 24, **characterized in that** the supersaturation with salts of the aqueous medium is a salt concentration which is greater than the natural solubility of these salts in the liquid medium.

**26.** Use according to claim 25, **characterized in that** the salt concentration is 5 times as high as the natural solubility of these salts in the liquid medium.

**27.** Use of the device according to any of the preceding claims 1 to 18, to bring about by addition of reagents to the treated liquid medium chemical reactions, precipitation reactions or crystallization reactions.

**28.** Method to neutralize, to remove and/or to prevent the growth of undifferentiated cells and/or microorganisms present in a liquid medium and/or to bring about a supersaturation with salts in a liquid medium, wherein gas microbubbles (5) having an average diameter of less than 1 mm and ultrasound (4) are emitted into a compartment (2) communicating with a reservoir (6) of the liquid medium to be treated and wherein said gas microbubbles (5) are emitted into the ultrasound (4) field emitted into the compartment (2) **characterized in that** the said ultrasound (4) are high frequency ultrasound having a frequency between 100 KHz and several MHz.

**29.** Method according to claim 28, **characterized in that** the liquid medium is an aqueous liquid medium.

**30.** Method according to any one of the preceding claims 28 to 29, **characterized in that** the gas microbubbles (5) are air or oxygen microbubbles.

**31.** Method according to claim 29, **characterized in**

**that** the aqueous liquid medium is selected from the group consisting of swimming pools, water reservoirs, aquariums or industrial cooling circuits.

32. Method according to claim 31, **characterized in that** the cooling industrial circuit is a cooling tower.

33. Method according to claim 29, **characterized in that** the aqueous liquid medium is a physiological liquid which can be administered to man or animal or extracted from man or animal.

34. Method according to claim 33, **characterized in that** the physiological liquid is selected from the group consisting of blood, serum or cerebrospinal fluid.

35. Method according to any of the preceding claims 28 to 34, **characterized in that** the average diameter of the gas microbubbles (5) is less than 50 $\mu$m.

36. Method according to any of the preceding claims 28 to 35, **characterized in that** the average diameter of the gas microbubbles (5) is less than 30 $\mu$m.

37. Method according to any of the preceding claims 28 to 36, **characterized in that** the ultrasound (4) emitted into the compartment (2) do not generate a stationary field phenomenon.

38. Method according to any of the preceding claims 28 to 37, **characterized in that** light whose electromagnetic radiation is mainly in the visible range is emitted into the ultrasound (4) field.

39. Method according to any of the preceding claims 28 to 38, **characterized in that** the aqueous liquid medium is subjected to an electromagnetic treatment in order to modify nucleation, formation and/or growth of salt crystals in the aqueous liquid medium.

40. Method according to any of the preceding claims 28 to 38, **characterized in that** said micro-organims are pathogenic or non-pathogenic microorganisms.

41. Method according to any of the preceding claims 28 to 40, **characterized in that** said microorganisms are selected from the group consisting of bacteria, viruses and/or algae.

42. Method according to any of the preceding claims 28 to 39, **characterized in that** the undifferentiated cells are cells presenting an hyperproliferative effect.

43. Method according to claim 42, **characterized in that** the cells presenting an hyperproliferative effect are selected from the group consisting of cancer cells, bone marrow cells or totipotent cells.

44. Method according to any of the preceding claims 28 to 43, **characterized in that** the supersaturation with salts in the aqueous liquid medium is a salt concentration which is greater than the natural solubility of these salts in the aqueous media.

45. Method according to claim 44, **characterized in that** the salt concentration is 5 times as high as the natural solubility of these salts in the aqueous media.

FIG.1

FIG.2

Fig.3

FIG.4

15

Fig.5

EP 0 912 445 B1